# DEMANDE DE BREVET EUROPEEN

(11) **EP 1 870 050 A2**
(43) Date de publication de la demande: **26.12.2007**
(21) Numéro de dépôt: 07110826.0
(22) Date de dépôt: 22.06.2007
(51) Int. Cl.: A61B 17/86

(54) **Ensemble chirurgical pour la reperation osseuse comprenant une vis cylindrique du type vis de Herbert**

(30) Priorité: 22.06.2006 FR 0605608
(71) Demandeur: Textile Hi-Tec (T.H.T.), 34220 Verreries de Moussans (FR)
(72) Inventeur: Houard, William, 81270, LABASTIDE-ROUAIROUX (FR)
(74) Mandataire: Hennion, Jean-Claude

(57) **Abrégé**

L'ensemble chirurgical pour la réparation osseuse comprend une broche métallique de guidage (8) et une vis cylindrique (1) présentant deux extrémités filetées (2,4), une portion intermédiaire lisse (6) et un trou central longitudinal (7) débouchant pour le passage de la broche de guidage (8).

Le filetage (5) de la seconde extrémité a un pas (P2) et un diamètre (D2) donnés, différents du pas (P1) et du diamètre (D1) du filetage de la première extrémité (2).

De plus il comporte un élément de comblement (9) apte à obturer sur au moins une partie de sa longueur le trou central longitudinal (7) après insertion de la vis (1) dans l'os et retrait de la broche métallique de guidage (8), lequel élément peut être une tige cylindrique (9), ayant un diamètre légèrement inférieur au diamètre du trou central (7), de préférence dans un matériau sécable, la vis (1) et l'élément de comblement peuvent être dans un matériau biodégradable et/ou résorbable.

## Description

La présente invention concerne le domaine chirurgical de la réparation osseuse. Elle concerne plus particulièrement un ensemble chirurgical comprenant une vis de compression du type vis de HERBERT.

On connaît par le document US.4.175.555, une vis de compression pour réparation osseuse, connue depuis lors sous l'appellation vis de HERBERT du nom de son inventeur. Cette vis cylindrique présente une première extrémité filetée dont le filetage a un pas et un diamètre donnés, une seconde extrémité filetée dont le filetage a un pas et un diamètre donnés différents du pas et du diamètre de la première extrémité filetée et, entre les deux extrémités, une portion lisse.

L'objectif est de créer dans la partie osseuse dans laquelle est vissée la vis de HERBERT une zone de compression due à la différence de pas du filetage entre les deux extrémités.

En pratique, le filetage de la seconde extrémité proximale de la vis a un pas qui est inférieur au pas du filetage de la première extrémité distale et a au contraire un diamètre qui est supérieur à celui du filetage de la première extrémité distale. Le praticien réalise tout d'abord dans la partie osseuse un premier trou taraudé ayant une profondeur donnée et un premier taraudage qui correspond au filetage de la première extrémité distale. Il réalise ensuite, dans ce premier trou taraudé, un second trou moins profond de diamètre supérieur dont il réalise le taraudage, second taraudage qui correspond au filetage de la seconde extrémité proximale. Il introduit la vis de HERBERT par sa première extrémité distale dans le trou ainsi formé, à double taraudage. La vis de HERBERT comporte sur sa face distale une encoche ou un évidement permettant son vissage. Le praticien fait pénétrer par vissage la vis de HERBERT de telle sorte que le filetage de la première extrémité distale se déplace en coopération avec le second taraudage du second trou tandis que le filetage de la seconde extrémité proximale se déplace en coopération avec le premier taraudage, du premier trou. Lorsque la vis de HERBERT pénètre dans la partie osseuse sur une distance donnée sous l'effet de l'entraînement du filetage de l'extrémité distale, cette même distance a été parcourue par l'extrémité proximale avec corrélativement un nombre de tours plus élevé du filetage de ladite extrémité proximale, qui a un pas plus court que celui de l'extrémité distale. Ceci provoque un effet de poussée de l'arrière vers l'avant, générateur d'une force de compression sur le matériau se trouvant entre l'extrémité distale et l'extrémité proximale. Bien sûr la vis de HERBERT est disposée de telle sorte que la portion lisse entre les deux extrémités filetées soit au niveau d'une zone de fracture. Ainsi la force de compression qui est exercée entre les deux dites extrémités tend à rapprocher les parois de ladite zone de fracture.

Dans le document US.4.175.555, il est prévu que la vis de HERBERT puisse être pourvue d'une lumière ou canule longitudinale, permettant d'utiliser un fil métallique de guidage toutes les fois où cela s'avère nécessaire, ledit fil métallique étant retiré une fois la vis de HERBERT implantée.

Il peut être souhaitable de retirer la vis de HERBERT lorsque la réparation osseuse est jugée satisfaisante. Cependant cette extraction peut s'avérer délicate voire problématique lorsque la vis comporte une lumière ou canule longitudinale dans la mesure où ce trou peut être le siège d'une certaine prolifération de cellules osseuses.

Le premier but de la présente invention est de proposer un ensemble chirurgical qui pallie cet inconvénient. Il s'agit d'un ensemble chirurgical pour la réparation osseuse qui comprend de manière connue par le document US.4.175.555 :
a) une broche métallique de guidage et
b) une vis cylindrique présentant :
   - une première extrémité filetée dont le filetage a un pas et un diamètre donnés,
   - une seconde extrémité filetée dont le filetage a un pas et un diamètre donnés, différents du pas et du diamètre de filetage de la première extrémité filetée,
   - une portion lisse entre les deux extrémités filetées et
   - un trou central longitudinal débouchant pour le passage de la broche métallique de guidage.

De manière caractéristique, selon la présente invention, l'ensemble chirurgical comporte un élément de comblement, apte à obturer sur au moins une partie de sa longueur le trou central longitudinal, après insertion de la vis dans l'os et retrait de la broche métallique de guidage.

L'élément de comblement étant une tige cylindrique, ayant un diamètre légèrement inférieur au diamètre du trou central, ceci permet outre la facilité de mise en oeuvre de constituer une vis présentant une haute tenue mécanique, notamment de résistance au cisaillement, à la flexion et à la rupture, la tige agissant dès son introduction comme un élément de renfort mécanique.

De préférence cette tige de comblement est dans un matériau sécable. Ainsi, il suffit au praticien, après insertion de la vis dans l'os et retrait de la broche métallique de guidage, d'introduire dans le trou central ladite tige sur toute la longueur de la vis et de la sectionner au ras de la seconde extrémité de la vis.

Selon une variante préférée de réalisation, la vis est dans un matériau biodégradable et/ou résorbable. Dans ce cas, de préférence, l'élément de comblement est également dans un matériau biodégradable et/ou résorbable.

Le matériau biodégradable et/ou résorbable dans lequel est formée la vis et éventuellement l'élément de comblement peut être avantageusement choisi dans le groupe suivant :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

Par ailleurs de préférence ledit matériau biodégradable et/ou résorbable comporte une charge, notamment en proportion en poids de 5 à 60 %, constituée d'une poudre ou d'une nano poudre choisie parmi les constituants suivants : hydroxy apatite et/ou de phosphate de calcium, notamment de β tricalcium phosphate, et/ou d'hydrogéno phosphate de calcium et/ou de carbonate de calcium et/ou d'hydrogéno carbonate de calcium.

Le matériau biodégradable et/ou résorbable est sélectionné en sorte que la perte des propriétés mécaniques de la vis et éventuellement de l'élément de comblement, en terme de force de rupture, est de l'ordre de 50 % au bout de six mois et quasi-totale au bout d'un an.

Selon un mode particulier de réalisation, la vis et l'élément de comblement sont tous deux dans des matériaux biodégradables et/ou résorbables qui sont de natures différentes, la perte des propriétés mécaniques de l'élément de comblement intervenant plus rapidement que celle de la vis. Cette disposition particulière permet d'obtenir une colonisation de cellules osseuses par l'intermédiaire de l'élément de comblement, à l'intérieur de la vis, ce qui s'avère préférable.

L'ensemble chirurgical caractérisé ci-dessus peut également comprendre un ancillaire de pose, comportant un tournevis, des forets et des tarauds adaptés au diamètre et au pas des filetage de la vis, lesdits tournevis, forets et tarauds étant percés d'un trou central longitudinal débouchant pour le passage de la broche métallique de guidage.

Ainsi le praticien a, à sa disposition, tous les équipements qui lui sont nécessaires pour la pose de la vis de compression, du type vis de HERBERT, munie conformément à l'invention, d'un élément de comblement du trou central longitudinal nécessaire au passage de la broche métallique de guidage.

La présente invention sera mieux comprise à la lecture de la description qui va être faite d'un exemple de réalisation d'un ensemble chirurgical comprenant une broche métallique de guidage, une vis de type HERBERT, avec un trou central longitudinal débouchant pour le passage d'une broche de guidage et un ancillaire de pose de ladite vis, illustré par le dessin annexé dans lequel :
- La figure 1 est une représentation schématique de côté d'une vis cylindrique contenant une tige de comblement,
- La figure 2 est une vue en perspective depuis l'extrémité arrière de la vis cylindrique et
- La figure 3 est une vue schématique en plan de l'ancillaire de pose, avec la broche métallique de guidage, le tournevis, deux forets et deux tarauds.

L'ensemble chirurgical de la présente invention est principalement destiné aux ostéosynthèses de petits fragments osseux tels que l'ostéotomie du scaphoïde carpien. Ceci n'est bien sûr pas limitatif des applications possibles de la présente invention.

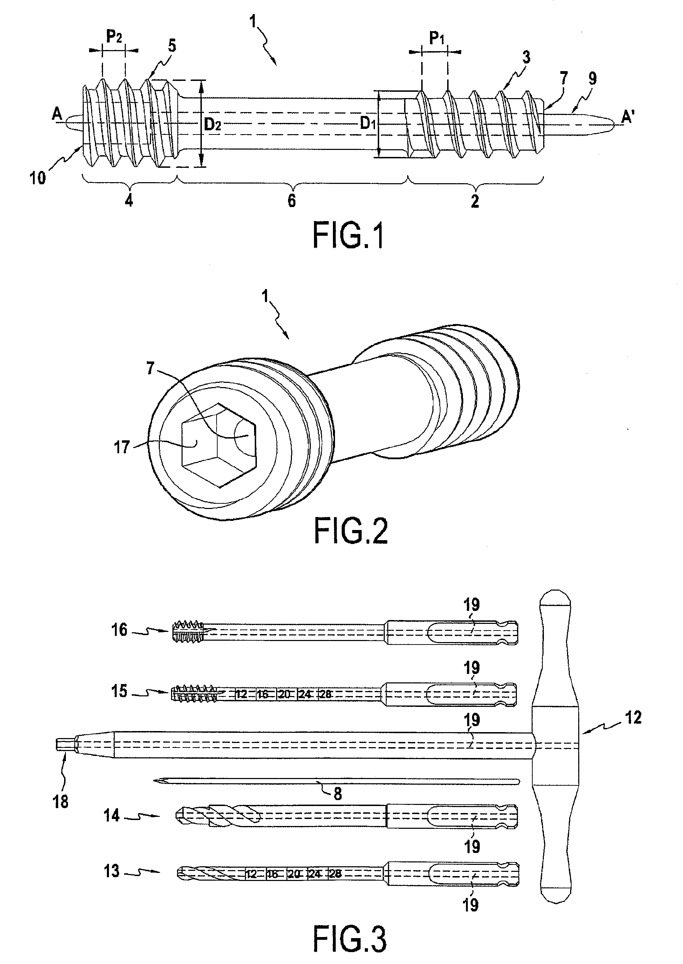

Cet ensemble chirurgical utilise, pour la réparation osseuse, une vis cylindrique dont la structure est connue sous la dénomination vis de HERBERT. Il s'agit d'une vis 1 à double filetage et à effet compressif. Plus précisément, en référence à la figure 1, ladite vis 1 présente :
- une première portion d'extrémité filetée 2, dont le filetage 3 a un pas P1 et un diamètre D1 donnés,
- une seconde portion d'extrémité filetée 4 dont le filetage 5 a un pas P2 et un diamètre D2 donnés, différents du pas P1 et du diamètre D1 de la première portion d'extrémité 2,
- une portion lisse 6 intermédiaire entre les deux portions d'extrémité 2, 4.

Lors de son implantation dans la partie osseuse, la première portion d'extrémité 2 constitue la portion distale, qui pénètre la première dans l'os, et la seconde portion 4 constitue la portion proximale.

La vis cylindrique 1 présente également un trou central longitudinal débouchant 7 (figure 2), ledit trou 7 étant destiné au passage d'une broche métallique de guidage 8 (figure 3).

Selon la présente invention, l'ensemble chirurgical comporte également un élément de comblement 9 apte à obturer sur au moins une partie de sa longueur le trou central 7 après insertion de la vis dans la partie osseuse et retrait de la broche métallique 8 de guidage. Cet élément de comblement est une tige de comblement 9 qui est cylindrique, avec un diamètre légèrement inférieur au diamètre du trou central 7, en sorte de pouvoir être introduite de manière sensiblement ajustée dans le trou central 7.

De préférence, cette tige de comblement 9 est sécable de telle sorte que le praticien, après implantation de la vis 1 et retrait de la broche métallique de guidage 8, puisse enfiler la tige de comblement 9 dans le trou central 7 ainsi libéré et sectionner ladite tige 9 à proximité immédiate de la face proximale 10 de la vis 1.

Le filetage 3 de la portion distale 2 de la vis 1 a un pas P1 supérieur au pas P2 du filetage 5 de la portion proximale 4. Au contraire, le filetage 3 de la première portion d'extrémité distale 2 de la vis 1 a un diamètre D1 qui est inférieur au diamètre D2 de la seconde portion d'extrémité proximale 4 de la vis 1.

Pour réaliser l'implantation dans l'os de la vis 1, le praticien met en oeuvre l'ancillaire de pose qui est illustré à la figure 3, en complément de la broche métallique de guidage 8.

Cet ancillaire de pose 11 comprend un tournevis 12, deux forets 13, 14 et deux tarauds 15, 16.

Tous ces instruments 12 à 16 sont tous percés d'un trou central 19 longitudinal débouchant, pour le passage de la broche métallique de guidage 8.

Le praticien procède de la manière suivante. Il place tout d'abord dans la partie osseuse dont on cherche à réaliser la réparation la broche métallique de guidage 8 jusqu'à la profondeur adéquate puis il réalise ensuite toutes les opérations de perçage et de taraudage en utilisant cette broche 8 comme élément de guidage. A l'aide du premier foret 13, il perce sur une profondeur donnée un alésage dont le diamètre correspond sensiblement au diamètre de la première portion proximale 2, non compris le filetage 3. A l'aide du premier taraud 15, il procède au taraudage de ce premier alésage. Ce taraudage est complémentaire du filetage 3 de la portion proximale 2. Ensuite le praticien perce, à l'aide du second foret 14, un second alésage sur une profondeur limitée, le diamètre de cet alésage correspondant sensiblement au diamètre de la portion distale 4 de la vis 1, non compris le filetage 5. Puis à l'aide du second taraud 16, il procède au taraudage de ce second alésage. Ceci étant fait, il peut enfin implanter la vis 1 en l'introduisant dans le premier alésage à partir de sa portion distale 2 et en la déplaçant dans le premier et le second alésages en la faisant pivoter sur elle-même selon son axe AA' à l'aide du tournevis 12.

Dans l'exemple illustré, dans la portion proximale 4, il est prévu une empreinte en creux, de configuration hexagonale 17, correspondant à l'extrémité hexagonale 18 du tournevis 12. Lors de ce pivotement, le filetage 3 de la portion distale 2 coopère avec le premier taraudage pour entraîner en déplacement vers l'avant la vis 1 le long de la broche métallique de guidage 8. Par ailleurs le filetage 5 de la portion proximale 4 coopère lui aussi avec le taraudage du second alésage. Du fait que le filetage 5 a un pas P2 qui est inférieur à celui P1 du filetage 3 de la portion distale 2, le déplacement vers l'avant de la portion proximale 4 est plus rapide que le déplacement vers l'avant de la portion distale 2 de telle sorte qu'on observe une poussée exercée par la portion proximale 4 vis-à-vis de la portion intermédiaire 6.

Le positionnement de la vis est réalisé par le praticien de telle sorte que la portion intermédiaire 6 de la vis 1 soit localisée au niveau de la zone de fracture. Ainsi la poussée exercée entre les deux portions proximale 4 et distale 2 permet de comprimer la zone de fracture et faciliter la réparation osseuse.

Une fois la vis implantée, le praticien retire la broche métallique de guidage 8 et introduit dans le trou central 7 de la vis 1, de manière sensiblement ajustée, la tige de comblement 9 en sorte d'obturer le trou central 7 et renforcer la vis en contrainte de cisaillement voire de flexion. Il peut en particulier sectionner l'extrémité de la tige 9 au ras de la face proximale 10 de la vis 1.

Dans un mode préféré de réalisation, la vis 1 et la tige de comblement 9 sont toutes deux biodégradables et/ou résorbables, étant formées dans un matériau ayant cette propriété. Il peut s'agir d'un matériau choisi parmi le groupe suivant :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

A titre d'exemple précis de réalisation pour une ostéosynthèse de petits fragments osseux tels que l'ostéotomie du scaphoïde carpien, la vis a une longueur de 16 ou 20 mm, avec chacune des deux portions filetées faisant sensiblement le quart de cette longueur. La tige de comblement 9 a un diamètre de l'ordre de 0,8 mm et une longueur de l'ordre de 50 mm. Elle est dans un matériau sécable qui est le même que celui dont est constituée la vis 1, à savoir en acide poly lactique.

Le matériau biodégradable et/ou résorbable comporte une charge se présentant sous forme de poudre permettant d'améliorer la reconstruction osseuse au cours de la résorption de la vis de la tige de comblement. Cette poudre ou nano poudre est de préférence choisie parmi les composants suivants : d'hydroxy apatite et/ou de phosphate de calcium, notamment de β tricalcium phosphate et/ou d'hydrogéno phosphate de calcium et/ou de carbonate de calcium et/ou d'hygéno carbonate de calcium, de préférence à raison de 5 à 60 % en poids de charge.

Le pourcentage de charge, qui peut être entre 5 et 60 % en poids, et le choix du matériau, déterminent l'évolution dans le temps de la perte des propriétés mécaniques de la vis et de l'élément de comblement en terme de force de rupture. Avantageusement, on fait en sorte que cette perte de propriétés mécaniques soit de l'ordre de 50% au bout de six mois et quasi-totale au bout d'un an.

Egalement, il est préférable que la perte des propriétés mécaniques de l'élément de comblement intervienne plus vite que celle de la vis. Ainsi, on obtient le démarrage de la colonisation des cellules osseuses par l'intérieur de la vis, au niveau de l'élément de comblement, ce qui permet d'obtenir une reconstruction osseuse beaucoup plus homogène.

## Revendications

1. Ensemble chirurgical pour la réparation osseuse comprenant :
a) une broche métallique de guidage (8) et
b) une vis cylindrique (1) présentant :
- une première extrémité filetée (2) dont le filetage (3) a un pas (P1) et un diamètre (D1) donnés,
- une seconde extrémité filetée (4) dont le filetage (5) a un pas (P2) et un diamètre (D2) donnés, différents du pas (P1) et du diamètre (D1) de filetage de la première extrémité filetée (2),
- une portion lisse (6) entre les deux extrémités filetées (2,4) et
- un trou central longitudinal (7) débouchant pour le passage de la broche métallique de guidage (8),
**caractérisé en ce qu'**il comporte un élément de comblement (9) apte à obturer sur au moins une partie de sa longueur le trou central longitudinal (7) après insertion de la vis (1) dans l'os et retrait de la broche métallique de guidage (8) et **en ce que** l'élément de comblement est une tige cylindrique (9), ayant un diamètre légèrement inférieur au diamètre du trou central (7).

2. Ensemble selon la revendication 1, **caractérisé en ce que** la tige de comblement (9) est dans un matériau sécable.

3. Ensemble selon l'une quelconque des revendications 1 et 2, **caractérisé en ce que** la vis (1) est dans un matériau biodégradable et/ou résorbable.

4. Ensemble selon la revendication 3, **caractérisé en ce que** l'élément de comblement (9) est dans un matériau biodégradable et/ou résorbable.

5. Ensemble selon l'une des revendications 3 ou 4, **caractérisé en ce que** le matériau biodégradable et/ou résorbable est choisi dans le groupe suivant :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

6. Ensemble selon la revendication 5, **caractérisé en ce que** ledit matériau biodégradable et/ou résorbable comporte une charge, notamment en proportion en poids de 5 à 60 %, constituée d'une poudre ou d'une nano poudre choisie parmi les constituants suivants : hydroxy apatite et/ou de phosphate de calcium, notamment de β tricalcium phosphate, et/ou d'hydrogéno phosphate de calcium et/ou de carbonate de calcium et/ou d'hydrogéno carbonate de calcium.

7. Ensemble selon l'une quelconque des revendications 3 à 6, **caractérisé en ce que** le matériau biodégradable et/ou résorbable est sélectionné en sorte que la perte des propriétés mécaniques de la vis et éventuellement de l'élément de comblement, en terme de force de rupture, est de l'ordre de 50 % au bout de six mois et quasi-totale au bout d'un an.

8. Ensemble selon la revendication 4, **caractérisé en ce que** la vis et l'élément de comblement sont tous deux dans des matériaux biodégradables et/ou résorbables qui sont de natures différentes, la perte des propriétés mécaniques de l'élément de comblement intervenant plus rapidement que celle de la vis.

9. Ensemble selon l'une quelconque des revendications 1 à 8 comprenant un ancillaire de pose, comportant un tournevis (12), des forets (13,14) et des tarauds (15,16) adaptés au diamètre (D1,D2) et au pas (P1,P2) des filetages (3,5) de la vis, lesdits tournevis (12), forets (13,14)et tarauds (15,16)étant percés d'un trou central longitudinal débouchant pour le passage de la broche métallique de guidage (8).
